# EUROPÄISCHE PATENTSCHRIFT

(11) **EP 1 373 265 B1**
(45) Veröffentlichungstag und Bekanntmachung des Hinweises auf die Patenterteilung: **06.05.2009**
(21) Anmeldenummer: 02714173.8
(22) Anmeldetag: 25.02.2002
(51) Int. Cl.: C07D 417/14, C07D 409/12, C07D 405/14, C07D 405/12, C07D 401/12

(54) **SUBSTITUIERTE 2, 6-DIAMINO-3, 5-DICYANO-4-ARYL-PYRIDINE UND IHRE VERWENDUNG ALS ADENOSINREZEPTOR SELEKTIVE LIGANDEN**
SUBSTITUTED 2,6-DIAMINO-3,5-DICYANO-4-ARYL-PYRIDINES AND THEIR USE AS ADENOSINE RECEPTOR-SELECTIVE LIGANDS
2, 6-DIAMINO-3, 5-DICYANO-4-ARYL-PYRIDINES SUBSTITUEES ET LEUR UTILISATION COMME LIGANDS SELECTEURS DU RECEPTEUR D'ADENOSINE

(30) Priorität: 07.03.2001 DE 10110747
(43) Veröffentlichungstag der Anmeldung: 02.01.2004
(73) Patentinhaber: Bayer HealthCare AG, 51368 Leverkusen (DE)
(72) Erfinder: ROSENTRETER, Ulrich, 42349 Wuppertal (DE); KRÄMER, Thomas, 42111 Wuppertal (DE); VAUPEL, Andrea, CH-4125 Riehen (CH); HÜBSCH, Walter, 42113 Wuppertal (DE); DIEDRICHS, Nicole, 42103 Wuppertal (DE); KRAHN, Thomas, 58135 Hagen (DE); DEMBOWSKY, Klaus, Boston, MA 02118 (US); STASCH, Johannes-Peter, 42651 Solingen (DE)
(86) Internationale Anmeldenummer: PCT/EP2002/001939
(87) Internationale Veröffentlichungsnummer: WO 2002/070520

(56) Entgegenhaltungen:
- EP-A- 0 705 820
- WO-A-01/25210
- WO-A-01/62233
- WO-A-02/06237
- WO-A-97/27177
- US-A- 4 052 510
- DATABASE CA [Online] CHEMICAL ABSTRACTS SERVICE, COLUMBUS, OHIO, US; QUINTELA, JOSE MARIA ET AL: "Reactivity of heterocyclic compounds. V. Behavior of 6-alkoxy-2-amino-(or chloro)-4-aryl-3,5-dicyanopyridines in the presence of nucleophiles" retrieved from STN Database accession no. 103:37345 XP002202945 & AN. QUIM., SER. C (1984), 80(3), 268-72 ,
- DATABASE CA [Online] CHEMICAL ABSTRACTS SERVICE, COLUMBUS, OHIO, US; CASTEDO, LUIS ET AL: "Synthesis and pharmacological activity of some nitrofuraldehyde cyanopyridine derivatives" retrieved from STN Database accession no. 103:37337 XP002202946 & EUR. J. MED. CHEM.--CHIM. THER. (1984), 19(6), 555-7 ,
- DATABASE CA [Online] CHEMICAL ABSTRACTS SERVICE, COLUMBUS, OHIO, US; FUENTES, LUIS ET AL: "Heterocycle synthesis. XVI. Reaction of malononitrile with benzylidenemalononitriles in presence of amines" retrieved from STN Database accession no. 94:139574 XP002202947 & AN. QUIM., SER. C (1980), 76(1), 68-9 ,

## Beschreibung

Die vorliegende Erfindung betrifft substituierte 2,6-Diamino-3,5-dicyano-4-arylpyridine, ein Verfahren zu ihrer Herstellung und ihre Verwendung als Arzneimittel.

Adenosin, ein Nucleosid aus Adenin und D-Ribose, ist ein endogener Faktor mit zellprotektiver Wirksamkeit, insbesondere unter zellschädigenden Bedingungen mit begrenzter Sauerstoff- und Substratversorgung, wie z.B. bei Ischämie in verschiedensten Organen (z.B. Herz und Gehirn).

Adenosin entsteht intrazellulär beim Abbau von Adenosin-5'-monophosphat (AMP) und S-Adenosylhomocystein als Zwischenprodukt, kann jedoch aus der Zelle freigesetzt werden und übt dann durch Bindung an spezifische Rezeptoren Funktionen als hormonähnliche Substanz oder Neurotransmitter aus.

Unter normoxischen Bedingungen ist die Konzentration des freien Adenosin im Extrazellulärraum sehr niedrig. Die extrazelluläre Konzentration von Adenosin erhöht sich in den betroffenen Organen jedoch dramatisch unter ischämischen bzw. hypoxischen Bedingungen. So ist beispielsweise bekannt, dass Adenosin die Thrombozyten-Aggregation hemmt und die Durchblutung der Herzkranzgefäße steigert. Weiterhin wirkt es auf die Herzfrequenz, auf die Ausschüttung von Neurotransmittern und auf die Lymphozyten-Differenzierung.

Diese Wirkungen von Adenosin zielen darauf ab, das Sauerstoffangebot der betroffenen Organe zu erhöhen bzw. den Stoffwechsel dieser Organe zu drosseln, um damit unter ischämischen oder hypoxischen Bedingungen eine Anpassung des Organstoffwechsels an die Organdurchblutung zu erreichen.

Die Wirkung von Adenosin wird über spezifische Rezeptoren vermittelt. Bekannt sind bisher die Subtypen A1, A2a, A2b und A3. Die Wirkungen dieser Adenosin-Rezeptoren werden intrazellulär durch den Botenstoff cAMP vermittelt. Im Falle der Bindung von Adenosin an die A2a- oder A2b-Rezeptoren kommt es über eine Aktivierung der membranständigen Adenylatzyklase zu einem Anstieg des intrazellulären cAMP, während die Bindung des Adenosin an die A1- oder A3-Rezeptoren über eine Hemmung der Adenylatzyklase eine Abnahme des intrazellulären cAMP-Gehalts bewirkt.

Als "Adenosinrezeptor-selektive Liganden" werden erfindungsgemäß solche Substanzen bezeichnet, die selektiv an einen oder mehrere Subtypen der Adenosinrezeptoren binden und dabei entweder die Wirkung des Adenosin nachahmen (Adenosin-Agonisten) oder dessen Wirkung blockieren (Adenosin-Antagonisten) können.

Adenosinrezeptor-selektive Liganden lassen sich nach ihrer Rezeptorselektivität in verschiedene Klassen einteilen, so z.B. in Liganden, die selektiv an die A1- oder die A2-Rezeptoren des Adenosin binden, bei letzteren auch beispielsweise solche, die selektiv an die A2a- oder die A2b-Rezeptoren des Adenosin binden. Auch sind Adenosinrezeptor-Liganden möglich, die selektiv an mehrere Subtypen der Adenosinrezeptoren binden, so z.B. Liganden, die selektiv an die A1- und an die A2-, jedoch nicht an die A3-Rezeptoren des Adenosin binden.

Die zuvor genannte Rezeptor-Selektivität lässt sich bestimmen durch die Wirkung der Substanzen an Zelllinien, die nach stabiler Transfektion mit der entsprechenden cDNA die jeweiligen Rezeptorsubtypen exprimieren (siehe hierzu die Druckschrift M.E. Olah, H. Ren, J. Ostrowski, K.A. Jacobson, G.L. Stiles, "Cloning, expression, and characterization of the unique bovine A1 adenosine receptor. Studies on the ligand binding site by site-directed mutagenesis." in J. Biol. Chem. 267 (1992) Seiten 10764-10770, deren Offenbarung hiermit im vollen Umfang durch Bezugnahme eingeschlossen ist).

Die Wirkung der Substanzen an solchen Zelllinien lässt sich erfassen durch biochemische Messung des intrazellulären Botenstoffes cAMP (siehe hierzu die Druckschrift K.N. Klotz, J. Hessling, J. Hegler, C. Owman, B. Kull, B.B. Fredholm, M.J. Lohse, "Comparative pharmacology of human adenosine receptor subtypes - characterization of stably transfected receptors in CHO cells" in Naunyn Schmiedebergs Arch. Pharmacol. 357 (1998) Seiten 1-9, deren Offenbarung hiermit im vollen Umfang durch Bezugnahme eingeschlossen ist).

Bei den aus dem Stand der Technik bekannten, als "adenosinrezeptor-spezifisch" geltenden Liganden handelt es sich überwiegend um Derivate auf Basis des natürlichen Adenosins (S.-A. Poulsen und R.J. Quinn, "Adenosine receptors: new opportunities for future drugs" in Bioorganic and Medicinal Chemistry 6 (1998) Seiten 619-641; K. J. Broadley, "Drugs modulating adenosine receptors as potential therapeutic agents for cardiovascular diseases" in Exp. Opin. Ther. Patents 10 (2000) Seiten 1669-1692). Die aus dem Stand der Technik bekannten Adenosin-Liganden haben jedoch meistens den Nachteil, dass sie nicht wirklich rezeptorspezifisch wirken, schwächer wirksam sind als das natürliche Adenosin oder nach oraler Applikation nur sehr schwach wirksam sind. Daher werden sie aufgrund der zuvor genannten Nachteile überwiegend nur für experimentelle Zwecke verwendet.

Aufgabe der vorliegenden Erfindung ist es, pharmakologisch aktive Substanzen aufzufinden oder bereitzustellen, die für die Prophylaxe und/oder Behandlung verschiedenster Erkrankungen, insbesondere Erkrankungen des Herz-Kreislauf-Systems (kardiovaskuläre Erkrankungen), geeignet sind und dabei vorzugsweise als Adenosinrezeptor-selektive Liganden wirken.

Gegenstand der vorliegenden Erfindung ist die Verwendung von Verbindungen der Formel (I), 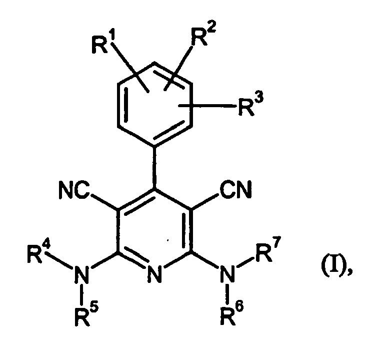 worin
- R¹: Wasserstoff, Hydroxy, Chlor, Nitro, Methyl, Methoxy, Ethoxy, n-Propoxy, Isopropoxy, n-Butoxy oder -NH-C(O)-CH₃ bedeuten, wobei die Alkoxyreste ihrerseits durch Hydroxy, Methoxy, Ethoxy, n-Propoxy, Isopropoxy, n-Butoxy, -O-C(O)-CH₃ oder Cyclopropyl substituiert sein können,
- R² und R³: Wasserstoff bedeuten,
- R⁴: Wasserstoff, Methyl, Ethyl, n-Propyl oder Isopropyl bedeutet, wobei die Alkylreste ihrerseits durch Pyridyl, Methoxy, Ethoxy, n-Propoxy, Isopropoxy, n-Butoxy oder ein- oder zweifach durch Hydroxy substituiert sein können,
- R⁵: Wasserstoff bedeutet,
- R⁶: Methyl, Ethyl n-Propyl, Isopropyl, wobei die Alkylreste ihrerseits ein- oder zweifach, unabhängig voneinander, durch Cyclopropyl, Phenyl, das seinerseits wiederum durch Fluor, Trifluormethyl oder Methoxy substituiert sein kann, Pyridyl, Furyl, Thienyl, Benzimidazolyl, Pyrrolidinonyl, N-Methyl-Pyrrolidinonyl, N-Methyl-Pyrrolidinyl, N-Ethyl-Pyrrolidinyl, N-Methyl-Imidazolidinonyl substituiert sein können, oder Cyclopropyl bedeutet
und
- R⁷: Wasserstoff bedeutet
und ihre Salze, Hydrate, Hydrate der Salze und Solvate, ausgenommen die verbindung 2-Amino-4-phenyl-6-[(phenylmethyl)amino]-3,5-pyridindicarbonitril.

Die Verbindungen der Formel (I) können in Abhängigkeit vom Substitutionsmuster in stereoisomeren Formen, die sich entweder wie Bild und Spiegelbild (Enantiomere) oder die sich nicht wie Bild und Spiegelbild (Diastereomere) verhalten, existieren. Die Erfindung betrifft sowohl die Enantiomeren oder Diastereomeren als auch deren jeweilige Mischungen. Die Racemformen lassen sich ebenso wie die Diastereomeren in bekannter Weise in die stereoisomer einheitlichen Bestandteile trennen. Gleichermaßen betrifft die vorliegende Erfindung auch die übrigen Tautomeren der Verbindungen der Formel (I).

Salze der Verbindungen der Formel (I) können physiologisch unbedenkliche Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Trifluoressigsäure, Essigsäure, Propionsäure, Milchsäure, Weinsäure, Zitronensäure, Fumarsäure, Maleinsäure oder Benzoesäure.

Als Salze können auch Salze mit üblichen Basen genannt werden, wie beispielsweise Alkalimetallsalze (z.B. Natrium- oder Kaliumsalze), Erdalkalisalze (z.B. Calcium- oder Magnesiumsalze) oder Ammoniumsalze, abgeleitet von Ammoniak oder organischen Aminen wie beispielsweise Diethylamin, Triethylamin, Ethyldiisopropylamin, Prokain, Dibenzylamin, N-Methylmorpholin, Dihydroabietylamin, 1-Ephenamin oder Methylpiperidin.

Als Hydrate bzw. Solvate werden erfindungsgemäß solche Formen der Verbindungen der Formel (I) bezeichnet, welche in festem oder flüssigem Zustand durch Hydratation mit Wasser oder Koordination mit Lösungsmittelmolekülen eine Molekül-Verbindung bzw. einen Komplex bilden. Beispiele für Hydrate sind Sesquihydrate, Monohydrate, Dihydrate oder Trihydrate. Gleichermaßen kommen auch die Hydrate bzw. Solvate von Salzen der erfindungsgemäßen Verbindungen in Betracht.

Außerdem existieren auch Prodrugs der erfindungsgemäßen Verbindungen. Als Prodrugs werden solche Formen der Verbindungen der Formel (I) bezeichnet, welche selbst biologisch aktiv oder inaktiv sein können, jedoch unter physiologischen Bedingungen in die entsprechende biologisch aktive Form überführt werden können (beispielsweise metabolisch oder solvolytisch).

Die vorliegende Erfindung betrifft weiterhin ein Verfahren zur Herstellung der Verbindungen der Formel (I), dadurch gekennzeichnet, dass man

Verbindungen der Formel (II) 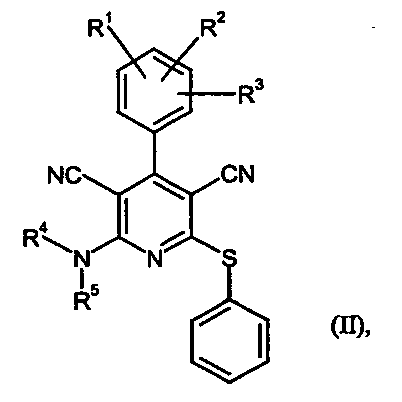 in welcher
R¹, R², R³, R⁴ und R⁵ die zuvor angegebene Bedeutung haben, in einem Lösemittel mit Verbindungen der Formel (III)

R⁶-NH-R⁷ (III),

in welcher
R⁶ und R⁷ die zuvor angegebene Bedeutung haben,
umsetzt.

Das erfindungsgemäße Verfahren kann durch das folgende Formelschema beispielhaft erläutert werden:

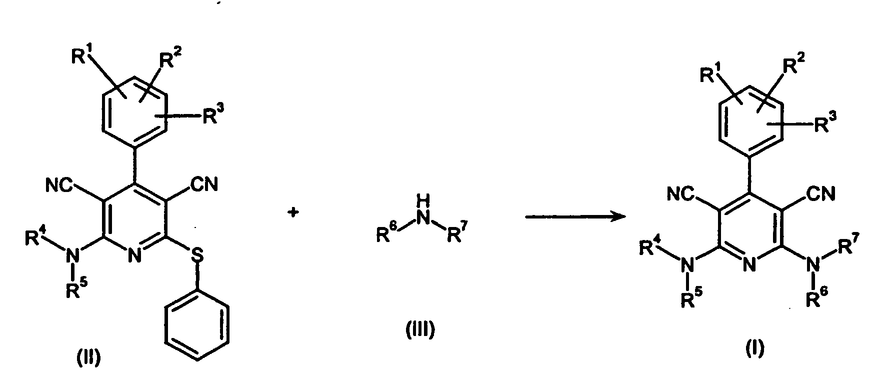

Als Lösemittel für das erfindungsgemäße Verfahren eignen sich organische Lösungsmittel, die sich unter den Reaktionsbedingungen nicht verändern. Hierzu gehören Alkohole wie Methanol, Ethanol und Isopropanol, Ketone wie Aceton und Methylethylketon, acyclische und cyclische Ether wie Diethylether und Tetrahydrofuran, Ester wie Essigsäureethylester oder Essigsäurebutylester, Kohlenwasserstoffe wie Benzol, Xylol, Toluol, Hexan oder Cyclohexan, chlorierte Kohlenwasserstoffe wie Dichlormethan, Chlorbenzol oder Dichlorethan oder andere Lösungsmittel wie Dimethylformamid, Acetonitril, Pyridin oder Dimethylsulfoxid (DMSO). Wasser ist als Lösungsmittel ebenso geeignet. Ebenso ist es möglich, Gemische der zuvor genannten Lösungsmittel einzusetzen. Bevorzugt ist Dimethylformamid.

Die Umsetzung erfolgt im allgemeinen in einem Verhältnis von 2 bis 8 Mol der Verbindung (III) auf ein Mol der Verbindung (II).

Die Reaktion erfolgt im allgemeinen in einem Temperaturbereich von -78°C bis +180°C, bevorzugt im Bereich von +20°C bis +160°C, insbesondere bei +80 bis +130°C.

Die Umsetzung kann bei normalem, erhöhtem oder erniedrigtem Druck durchgeführt werden (z.B. im Bereich von 0,5 bis 5 bar). Im allgemeinen arbeitet man bei Normaldruck.

Verbindungen der Formel (II), in denen R⁴ gleich R⁵ gleich Wasserstoff ist, sind dem Fachmann bekannt oder nach üblichen, literaturbekannten Methoden herstellbar [so z.B. Kambe et al., Synthesis 1981, Seiten 531-533; Elnagdi et al., Z. Naturforsch. 47b, Seiten 572 - 578 , (1991)]

Verbindungen der Formel (II), in denen R⁴ und /oder R⁵ ungleich Wasserstoff sind, können hergestellt werden, indem man

Verbindungen der Formel (IV) 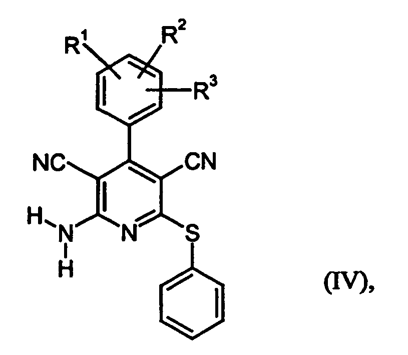 in welcher
R¹, R² und R³ die oben angegebenen Bedeutungen haben,
mit Kupfer(II)chlorid und Isoamylnitrit in einem geeigneten Lösungsmittel in Verbindungen der Formel (V), 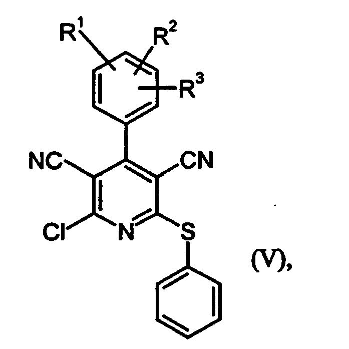 in welcher
R¹, R² und R³ die oben angegebenen Bedeutungen haben,
überführt,
diese anschließend mit Verbindungen der Formel (VI),

R⁴-NH-R⁵ (VI),

in welcher
R⁴ und R⁵ die oben angegebenen Bedeutungen haben,
zu Verbindungen der Formel (II) umsetzt.

Die Herstellung von Verbindungen der Formel (II) kann durch das folgende Formelschema beispielhaft erläutert werden:

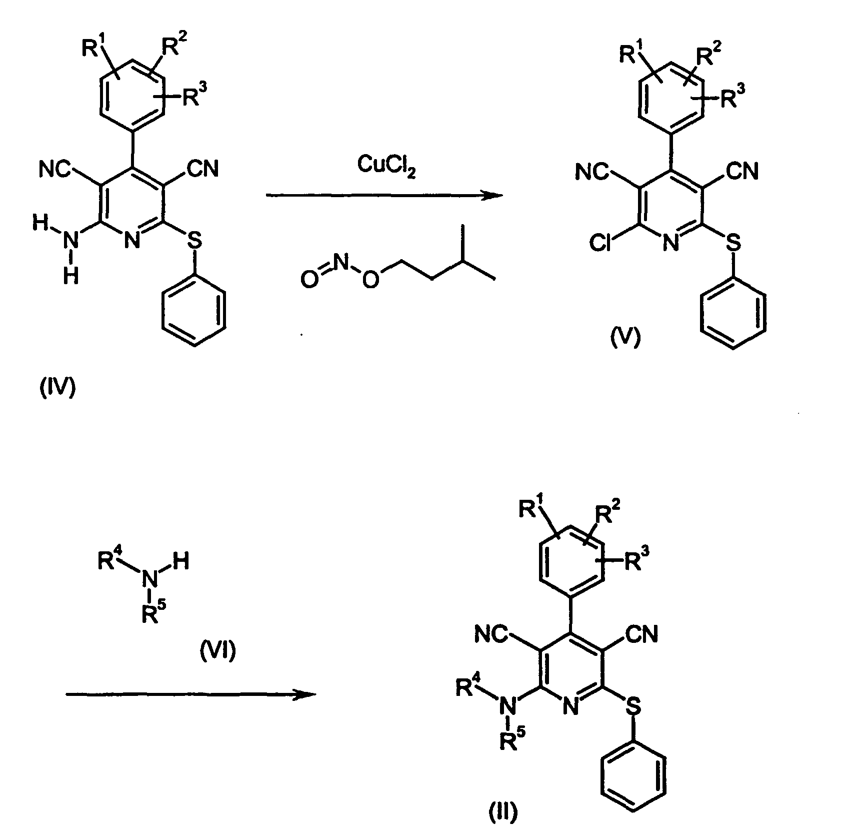

Der Verfahrensschritt (IV) → (V) erfolgt im allgemeinen mit einem Molverhältnis von 2 bis 12 Mol Kupfer(II)chlorid und 2 bis 12 Mol Isoamylnitrit auf ein Mol (IV).

Als Lösemittel für diesen Verfahrensschritt eignen sich alle organischen Lösemittel, die unter den Reaktionsbedingungen inert sind. Hierzu gehören, acyclische und cyclische Ether wie Diethylether und Tetrahydrofuran, Ester wie Essigsäureethylester oder Essigsäurebutylester, Kohlenwasserstoffe wie Benzol, Xylol, Toluol, Hexan oder Cyclohexan, chlorierte Kohlenwasserstoffe wie Dichlormethan, Chlorbenzol oder Dichlorethan oder andere Lösungsmittel wie Dimethylformamid, Acetonitril oder Pyridin. Ebenso ist es möglich, Gemische der zuvor genannten Lösemittel einzusetzen. Bevorzugte Lösemittel sind Acetonitril und Dimethylformamid.

Die Reaktion erfolgt im allgemeinen in einem Temperaturbereich von -78°C bis +180°C, bevorzugt im Bereich von +20°C bis +100°C, insbesondere bei +20 bis +60°C.

Die Umsetzung kann bei normalem, erhöhtem oder erniedrigtem Druck durchgeführt werden (z.B. im Bereich von 0,5 bis 5 bar). Im allgemeinen arbeitet man bei Normaldruck.

Der Verfahrensschritt (V) + (VI) → (II) erfolgt im allgemeinen mit einem Molverhältnis von 1 bis 8 Mol (VI) auf ein Mol (V).

Als Lösemittel eignen sich alle organischen Lösemittel, die unter den Reaktionsbedingungen inert sind. Hierzu gehören, Alkohole wie Methanol, Ethanol und Isopropanol, Ketone wie Aceton und Methylethylketon, acyclische und cyclische Ether wie Diethylether und Tetrahydrofuran, Ester wie Essigsäureethylester oder Essigsäurebutylester, Kohlenwasserstoffe wie Benzol, Xylol, Toluol, Hexan oder Cyclohexan, chlorierte Kohlenwasserstoffe wie Dichlormethan, Chlorbenzol oder Dichlorethan oder andere Lösungsmittel wie Dimethylformamid, Acetonitril, Pyridin oder Dimethylsulfoxid (DMSO). Wasser ist als Lösemittel ebenso geeignet. Ebenso ist es möglich, Gemische der zuvor genannten Lösemittel einzusetzen. Bevorzugtes Lösemittel ist Dimethylformamid.

Die Reaktion erfolgt im allgemeinen in einem Temperaturbereich von -78°C bis +180°C, bevorzugt im Bereich von +20°C bis +160°C, insbesondere bei +20 bis +40°C.

Die Umsetzung kann bei normalem, erhöhtem oder erniedrigtem Druck durchgeführt werden (z.B. im Bereich von 0,5 bis 5 bar). Im allgemeinen arbeitet man bei Normaldruck.

Verbindungen der Formel (IV) sind dem Fachmann bekannt oder nach üblichen, literaturbekannten Methoden herstellbar. Insbesondere kann auf die folgenden Druckschriften verwiesen werden, deren jeweiliger Inhalt durch Bezugnahme eingeschlossen wird:
- Kambe et al., Synthesis 1981, Seiten 531-533
- Elnagdi et al., Z. Naturforsch. 47b, Seiten 572 - 578, (1991)

Die Verbindungen der allgemeinen Formeln (III) und (VI) sind entweder kommerziell erhältlich, dem Fachmann bekannt oder nach üblichen Methoden herstellbar.

Überraschenderweise zeigen die Verbindungen der allgemeinen Formel (I) ein nicht vorhersehbares, wertvolles pharmakologisches Wirkspektrum und sind daher insbesondere zur Prophylaxe und/oder Behandlung von Erkrankungen geeignet.

Die Verbindungen der Formel (I) sind zur Prophylaxe und/oder Behandlung einer ganzen Reihe von Erkrankungen geeignet, so beispielsweise insbesondere von Erkrankungen des Herzkreislaufsystems (kardiovaskulären Erkrankungen).

Im Sinne der vorliegenden Erfindung sind unter Erkrankungen des Herz-Kreislauf-Systems bzw. kardiovaskulären Erkrankungen beispielsweise insbesondere die folgenden Erkrankungen zu verstehen: Koronare Herzkrankheit, Hypertonie (Bluthochdruck), Restenose wie z.B. Restenose nach Ballondilatation von peripheren Blutgefäßen, Arteriosklerose, Tachykardien, Arrhythmien, periphere und kardiale Gefäßerkrankungen, stabile und instabile Angina pectoris und Vorhofflimmern.

Weiterhin eignen sich die Verbindungen der Formel (I) beispielsweise insbesondere auch zur Reduktion des von einem Infarkt betroffenen Myokardbereichs.

Des weiteren eignen sich die Verbindungen der Formel (I) beispielsweise insbesondere zur Prophylaxe und/oder Behandlung von thromboembolischen Erkrankungen und Ischämien wie Myokardinfarkt, Hirnschlag und transitorischen ischämischen Attacken.

Schließlich kommen die Verbindungen der Formel (I) beispielsweise insbesondere auch für die Prophylaxe und/oder Behandlung von Diabetes, insbesondere Diabetes mellitus, in Betracht.

Die vorliegende Erfindung betrifft auch die Verwendung der Substanzen der Formel (I) zur Herstellung von Arzneimitteln und pharmazeutischen Zusammensetzungen zur Prophylaxe und/oder Behandlung der zuvor genannten Krankheitsbilder.

Die pharmazeutische Wirksamkeit der zuvor genannten Verbindungen der Formel (I) lässt sich insbesondere durch ihre Wirkung als selektive Liganden an A1-Adenosinrezeptoren erklären.

Als "selektiv" werden im Rahmen der vorliegenden Erfindung solche Adenosinrezeptor-Liganden bezeichnet, bei denen einerseits eine deutliche Wirkung an einem oder mehreren Adenosin-Rezeptor-Subtypen und andererseits keine oder eine deutlich schwächere Wirkung an einem oder mehreren anderen Adenosinrezeptor-Subtypen zu beobachten ist, wobei bezüglich der Testmethoden für die Wirk-Selektivität Bezug genommen wird auf die im Abschnitt A. II. beschriebenen Testmethoden.

Ein Vorteil der erfindungsgemäßen Verbindungen der Formel (I) ist, dass sie gegenüber Adenosinrezeptor-Liganden des Standes der Technik selektiver wirken.

Die Rezeptorselektivität kann bestimmt werden durch die biochemische Messung des intrazellulären Botenstoffes cAMP in den transfizierten Zellen, die spezifisch nur einen Subtyp der Adenosinrezeptoren exprimieren. Im Falle von Al-Agonisten (Kopplung bevorzugt über Gi-Proteine) wird dabei eine Abnahme des intrazellulären cAMP-Gehaltes festgestellt, unter Bedingungen bei denen die intrazelluläre cAMP Konzentration durch Stimulation der Adenylatzyklase deutlich erhöht würde. Im Falle von Al-Antagonisten wird dagegen ein Anstieg des intrazellulären cAMP-Gehaltes nach vergleichbarer Vorstimulation der Adenylatzyklase plus Stimulation mit Adenosin oder Adenosin ähnlichen Substanzen beobachtet.

So eignen sich Verbindungen der Formel (I), die selektiv an Adenosin-A1-Rezeptoren binden, bevorzugt zur Myokard-Protektion und zur Prophylaxe und/oder Behandlung von Tachykardien, Vorhof-Arrhythmien, Herzinsuffizienz, Herzinfarkt, akutem Nierenversagen, Diabetes, Schmerzzuständen sowie zur Wundheilung.

Weiterer Gegenstand der vorliegenden Erfindung sind Arzneimittel und pharmazeutische Zusammensetzungen, die mindestens eine Verbindung der Formel (I), vorzugsweise zusammen mit einem oder mehreren pharmakologisch unbedenklichen Hilfs- oder Trägerstoffen enthalten, sowie deren Verwendung zu den zuvor genannten Zwecken.

Für die Applikation der Verbindungen der Formel (I) kommen alle üblichen Applikationsformen in Betracht, d.h. also oral, parenteral, inhalativ, nasal, sublingual, rektal, lokal, wie z.B. bei Implantaten oder Stents, oder äußerlich wie z.B. transdermal. Bei der parenteralen Applikation sind insbesondere intravenöse, intramuskuläre, subkutane Applikation zu nennen, z.B. als subkutanes Depot. Besonders bevorzugt ist die orale Applikation.

Hierbei können die Wirkstoffe allein oder in Form von Zubereitungen verabreicht werden. Für die orale Applikation eignen sich als Zubereitungen u.a. Tabletten, Kapseln, Pellets, Dragees, Pillen, Granulate, feste und flüssige Aerosole, Sirupe, Emulsionen, Suspensionen und Lösungen. Hierbei muss der Wirkstoff in einer solchen Menge vorliegen, dass eine therapeutische Wirkung erzielt wird. Im allgemeinen kann der Wirkstoff in einer Konzentration von 0,1 bis 100 Gew.-%, insbesondere 0,5 bis 90 Gew.-%, vorzugsweise 5 bis 80 Gew.-%, vorliegen, d.h. der Wirkstoff sollte in Mengen vorliegen, die ausreichend sind, den angegebenen Dosierungsspielraum zu erreichen.

Zu diesem Zweck können die Wirkstoffe in an sich bekannter Weise in die üblichen Zubereitungen überführt werden. Dies geschieht unter Verwendung inerter, nichttoxischer, pharmazeutisch geeigneter Trägerstoffe, Hilfsstoffe, Lösungsmittel, Vehikel, Emulgatoren und/oder Dispergiermittel.

Als Hilfsstoffe seien beispielsweise aufgeführt: Wasser, nichttoxische organische Lösungsmittel wie z.B. Paraffine, pflanzliche Öle (z.B. Sesamöl), Alkohole (z.B. Ethanol, Glycerin), Glykole (z.B. Polyethylenglykol), feste Trägerstoffe wie natürliche oder synthetische Gesteinsmehle (z.B. Talkum oder Silikate), Zucker (z.B. Milchzucker), Emulgiermittel, Dispergiermittel (z.B. Polyvinylpyrrolidon) und Gleitmittel (z.B. Magnesiumsulfat).

Im Falle der oralen Applikation können Tabletten auch allgemein übliche Zusätze wie Natriumcitrat zusammen mit Zuschlagstoffen wie Stärke, Gelatine und dergleichen enthalten. Wässrige Zubereitungen für die orale Applikation können weiterhin mit Geschmacksaufbesserem oder Farbstoffen versetzt werden.

Im allgemeinen hat es sich als vorteilhaft erwiesen, bei parenteraler Applikation Mengen von etwa 0,1 bis etwa 10.000 µg/kg, vorzugsweise etwa 1 bis etwa 1.000 µg/kg, insbesondere etwa 1 µg/kg bis etwa 100 µg/kg Körpergewicht, zur Erzielung wirksamer Ergebnisse zu verabreichen. Bei oraler Applikation beträgt die Menge etwa 0,1 bis etwa 10 mg/kg, vorzugsweise etwa 0,5 bis etwa 5 mg/kg, insbesondere etwa 1 bis etwa 4 mg/kg Körpergewicht.

In Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt, kann es gegebenenfalls erforderlich sein, von den genannten Mengen abzuweichen.

Die vorliegende Erfindung wird an den folgenden Beispielen veranschaulicht, die die Erfindung jedoch keinesfalls beschränken.

### A. Bewertung der physiologischen Wirksamkeit

### I. Nachweis der kardiovaskulären Wirkung

### Langendorff-Herz der Ratte:

Narkotisierten Ratten wird nach Eröffnung des Brustkorbes das Herz entnommen und in eine konventionelle Langendorff-Apparatur eingeführt. Die Koronararterien werden volumenkonstant (10 ml/min) perfundiert und der dabei auftretende Perfusionsdruck wird über einen entsprechenden Druckaufnehmer registriert. Eine Abnahme des Perfusionsdrucks in dieser Anordnung entspricht einer Relaxation der Koronararterien. Gleichzeitig wird über einen in die linke Herzkammer eingeführten Ballon und einen weiteren Druckaufnehmer der Druck gemessen, der vom Herzen während jeder Kontraktion entwickelt wird. Die Frequenz des isoliert schlagenden Herzens wird rechnerisch aus der Anzahl der Kontraktionen pro Zeiteinheit ermittelt.

### II. Nachweis der Rezeptorselektivität

### a) Adenosin-A1-, A2a-, A2b- und A3-Rezeptorselektivität

Zellen der permanenten Linie CHO (Chinese Hamster Ovary) werden stabil mit der cDNA für die Adenosin-Rezeptor-Subtypen A1, A2a, A2b und A3 transfiziert. Die Bindung der Substanzen an die A2a- oder A2b-Rezeptorsubtypen wird bestimmt durch Messung des intrazellulären cAMP-Gehaltes in diesen Zellen mit einem konventionellen radioimmunologischen Assay (cAMP-RIA).

Im Falle der Wirkung der Substanzen als Agonisten kommt es als Ausdruck der Bindung der Substanzen zu einem Anstieg des intrazellulären cAMP-Gehaltes. Als Referenzverbindung dient in diesen Experimenten die Adenosin-analoge Verbindung NECA (5-N-Ethylcarboxamido-adenosin), die nicht selektiv, aber mit hoher Affinität an alle Adenosin-Rezeptor-Subtypen bindet und eine agonistische Wirkung besitzt (Klotz, K.N., Hessling, J., Hegler, J., Owman, C., Kull, B., Fredholm, B.B., Lohse, M.J., Comparative pharmacology of human adenosine receptor subtypes - characterization of stably transfected receptors in CHO cells, Naunyn Schmiedebergs Arch Pharmacol, 357 (1998), 1-9).

Die Adenosin-Rezeptoren A1 und A3 sind an ein Gi-Protein gekoppelt, d.h. eine Stimulation dieser Rezeptoren führt zu einer Inhibition der Adenylatcyclase und somit zu einer Senkung des intrazellulären cAMP-Spiegels. Zur Identifizierung von A1/A3-Rezeptor-Agonisten wird die Adenylatcyclase mit Forskolin stimuliert. Eine zusätzliche Stimulation der A1/A3-Rezeptoren hemmt jedoch die Adenylatcyclase, so dass A1/A3-Rezeptor-Agonisten über einen vergleichsweise geringen Gehalt der Zelle an cAMP detektiert werden können.

Für den Nachweis einer antagonistischen Wirkung an Adenosin-Rezeptoren werden die mit dem entsprechenden Rezeptor transfizierten, rekombinanten Zellen mit NECA vorstimuliert und die Wirkung der Substanzen auf eine Reduktion des intrazellulären cAMP-Gehalts durch diese Vorstimulation untersucht. Als Referenzverbindung dient in diesen Experimenten XAC (xanthine amine congener), die nicht selektiv, aber mit hoher Affinität an alle Adenosinrezeptor-Subtypen bindet und eine antagonistische Wirkung besitzt (Müller, C.E., Stein, B., Adenosine receptor antagonists: structures and potential therapeutic applications, Current Pharmaceutical Design, 2 (1996), 501-530).

### b) Adenosin-A1-, A2a-, A2b- Rezeptorselektivität

Zellen der permanenten Linie CHO (Chinese Hamster Ovary) werden stabil mit der cDNA für die Adenosin-Rezeptor-Subtypen A1, A2a, A2b transfiziert. Die Adenosin A1 Rezeptoren sind über Gᵢ-Proteine und die Adenosin A2a und A2b Rezeptoren über Gs-Proteine an die Adenylatcyclase gekoppelt. Entsprechend wird die cAMP-Bildung in der Zelle inhibiert bzw. stimuliert. Über einen cAMP-abhängigen Promotor wird danach die Expression der Luziferase moduliert. Der Luciferase-Test wird mit dem Ziel hoher Sensitivität und Reproduzierbarkeit, geringer Varianz und guter Eignung für die Durchführung auf einem Robotersystem optimiert durch Variation mehrerer Testparameter, wie z.B. Zelldichte, Dauer der Anzuchtphase und der Testinkubation, Forskolin-Konzentration, Medium-Zusammensetzung. Zur pharmakologischen Charakterisierung der Zellen und zum Roboter-gestützten Substanztest-Screening wird das folgende Testprotokoll verwendet:

Die Stammkulturen wird in DMEM/F12 Medium mit 10% FCS (fötales Kälberserum) bei 37°C unter 5% CO₂ gezüchtet und jeweils nach 2-3 Tagen 1:10 gesplittet. Testkulturen werden von 1000 bis 3000 Zellen pro Napf in 384-well Platten ausgesät und ca. 48 Stunden bei 37°C angezogen. Dann wird das Medium durch eine physiologische Kochsalzlösung (130 mM NaCl, 5 mM KCl, 2 mM CaCl₂, 20 mM HEPES, 1 mM MgCl₂•6H₂O, 5 mM NaHCO₃, pH 7,4) ersetzt. Die in DMSO gelösten Substanzen werden 3 mal 1:10 mit dieser physiologischen Kochsalzlösung verdünnt und zu den Testkulturen pipettiert (maximale DMSO-Endkonzentration im Testansatz: 0,5%) So erhält man Substanzendkonzentrationen von beispielsweise 5 µM bis 5 nM. 10 Minuten später wird Forskolin zu den A1 Zellen zugegeben und anschließend werden alle Kulturen für 4 Stunden bei 37°C inkubiert. Danach wird zu den Testkulturen 35 µl Lösung, bestehend zu 50 % aus Lysereagenz (30 mM di-Natriumhydrogenphosphat, 10 % Glycerin, 3 % TritonX100, 25 mM TrisHCl, 2 mM Dithiotreitol (DTT), pH 7,8) und zu 50% aus Luciferase Substrat Lösung (2,5 mM ATP, 0,5 mM Luciferin, 0,1 mM Coenzym A, 10 mM Tricin, 1,35 mM MgSO₄, 15 mM DTT, pH 7,8) zugegeben, ca. 1 Minute geschüttelt und die Luciferase-Aktivität mit einem Kamerasystem gemessen.

### B. Ausführungsbeispiele

### Beispiel 1

### 2-Amino-4-(4-hydroxyphenyl)-6-[(3-pyridinylmethyl)amino]-3,5-pyridindicarbonitril-trifluoracetat

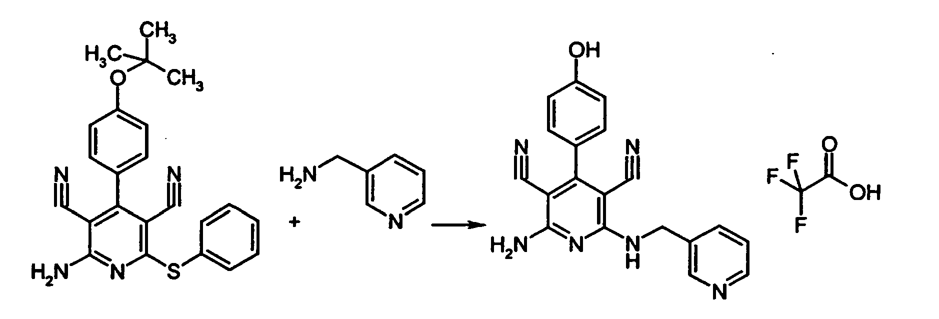

80,1 mg (0,2 mmol) 2-Amino-4-(4-tert-butoxyphenyl)-6-(phenylsulfanyl)-3,5-pyridindicarbonitril werden zusammen mit 86 mg (0,8 mmol) 3-Picolylamin in 0,5 ml Dimethylsulfoxid (DMSO) 10 Stunden bei 120°C geschüttelt. Die Reaktionslösung wird durch präparative HPLC gereinigt.
Säule: Grom-Sil 120 ODS-4 HE 5µm 20x50 mm,
Vorsäule: Gromsil ODS 4 HE 15µm 10x20 mm.
Flußrate: 25 ml/min.
Gradient (A = Acetonitril, B = Wasser + 0.3% Trifluoressigsäure):

| | |
|---|---|
| 0 min | 10%A; |
| 2.00 min | 10% A ; |
| 6.00 min | 90% A ; |
| 7.00 min | 90% A ; |
| 7.10 min | 10% A ; |
| 8 min | 10% A. |

Detektion: 220 nm. Injektionsvolumen: 510 µl DMSO-Lsg.

Die Produktfraktion wird im Vakuum eingedampft und in 0,7 ml Trifluoressigsäure gelöst. Nach 30 min bei RT wird im Vakuum eingedampft.
Ausbeute: 31,3 mg (= 45,7 % d.Th.) Produkt
Massenspektrum: gesuchte Molmasse: 342, gefunden [M+H]⁺=343

### Beispiel 2

### 2-Amino-4-phenyl-6-[(3-pyridinylinethyl)amino]-3,5-pyridindicarbonitriltrifluoracetat

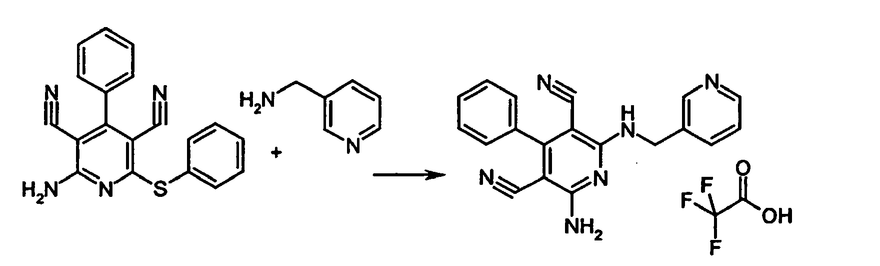

32,4 mg (0,1 mmol) 2-Amino-4-phenyl-6-(phenylsulfanyl)-3,5-pyridindicarbonitril werden zusammen mit 43 mg (0,4 mmol) 3-Picolylamin in 0,5 ml DMSO 18 Stunden bei 120°C geschüttelt. Die Reaktionslösung wird durch präparative HPLC gereinigt.
Säule: Grom-Sil 120 ODS-4 HE 5µm 20x50 mm,
Vorsäule: Gromsil ODS 4 HE 15µm 10x20 mm.
Flußrate: 25 ml/min.
Gradient (A = Acetonitril, B=Wasser + 0.3% Trifluoressigsäure):

| | |
|---|---|
| 0 min | 10% A ; |
| 2.00 min | 10% A ; |
| 6.00 min | 90% A ; |
| 7.00 min | 90% A ; |
| 7.10 min | 10% A ; |
| 8 min | 10% A. |

Detektion: 220 nm. Injektionsvolumen: 510 µl DMSO-Lsg.

Die Produktfraktion wird im Vakuum eingedampft.
Ausbeute: 21,5 mg (= 66 % d.Th.) Produkt
Massenspektrum: gesuchte Molmasse: 326, gefunden [M+H]⁺=327
¹H-NMR-Spektrum [DMSO-d₆]: δ = 4,55 [2H] d; 7,3 - 7,55 [8H] m; 7,95 [1H] s breit; 8,1 [1H] s breit; 8,5 [1H] s; 8,7 [1H] s.

Die in der folgenden Tabelle aufgeführten Verbindungen (Beispiel 3 bis 40) werden analog zu den zuvor aufgeführten Vorschriften der Beispiel 1 und 2 hergestellt. Die Identität und Reinheit der Verbindungen wird durch LC-MS nachgewiesen. Die Beispiele Nr. 7,8,11,25,37 und 60-62 sind Vergleichsbeispiele.

| **Bsp.- Nr.** | **Zielstruktur** | **gesuchte Molmasse** | **gefunden [M+H]⁺** | **Ausbeute (% d.Th.)** |
|---|---|---|---|---|
| 3 | 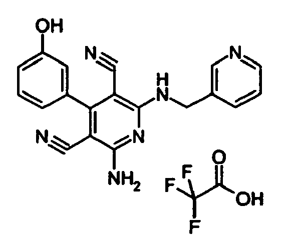 | 342 | 343 | 90 |
| 4 | 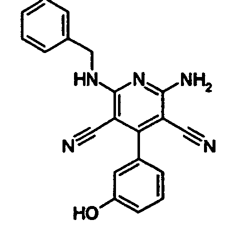 | 341 | 342 | 66 |
| 5 | 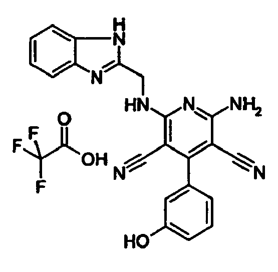 | 381 | 382 | 62 |
| 6 | 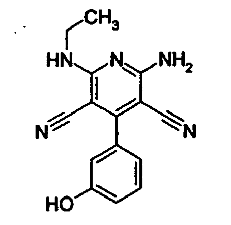 | 279 | 280 | 42 |
| 7 | 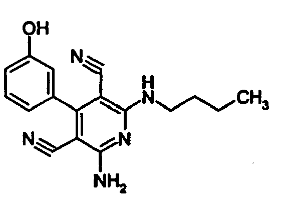 | 307 | 308 | 60 |
| 8 | 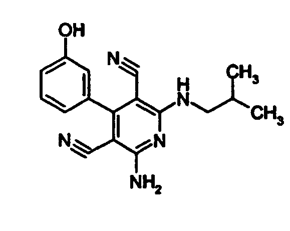 | 307 | 308 | 61 |
| 9 | 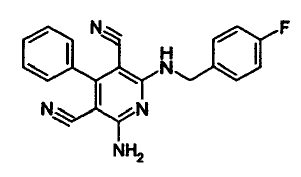 | 343 | 344 | 47 |
| 10 | 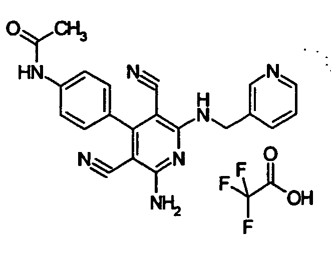 | 383 | 384 | 78 |
| 11 | 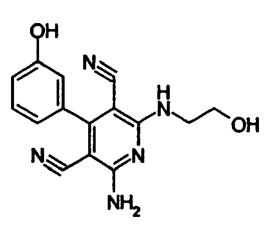 | 295 | 296 | 86 |
| 12 | 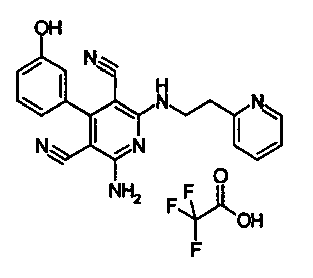 | 356 | 357 | 86 |
| 13 | 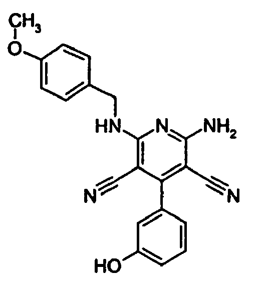 | 371 | 372 | 51 |
| 14 | 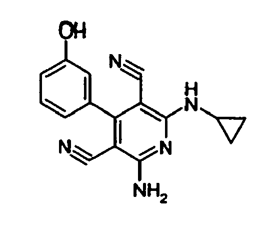 | 291 | 292 | 71 |
| 15 | 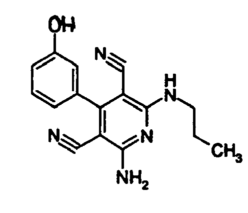 | 293 | 294 | 87 |
| 16 | 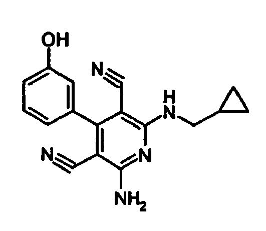 | 305 | 306 | 89 |
| 17 | 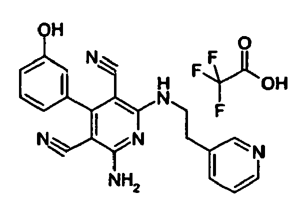 | 356 | 357 | 99 |
| 18 | 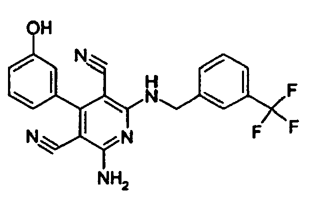 | 409 | 410 | 84 |
| 19 | 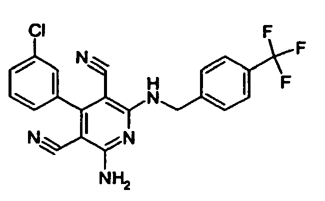 | 428 | 429 | 85 |
| 20 | 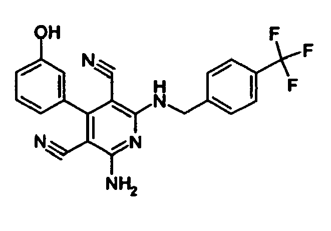 | 409 | 410 | 74 |
| 21 | 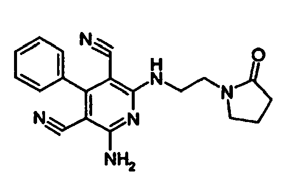 | 346 | 347 | 42 |
| 22 | 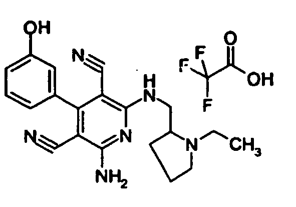 | 362 | 363 | quantitativ |
| 23 | 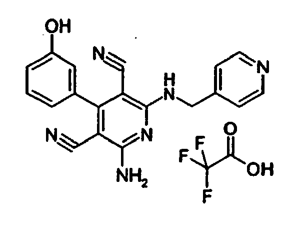 | 342 | 343 | 77 |
| 24 | 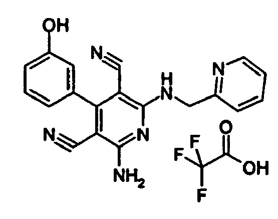 | 342 | 343 | quantitativ |
| 25 | 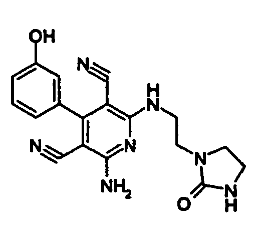 | 363 | 364 | quantitativ |
| 26 | 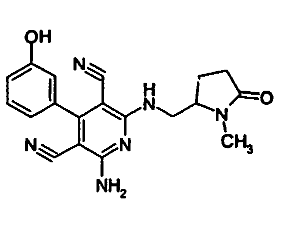 | 362 | 363 | 87 |
| 27 | 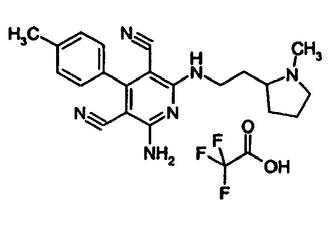 | 360 | 361 | 97 |
| 28 | 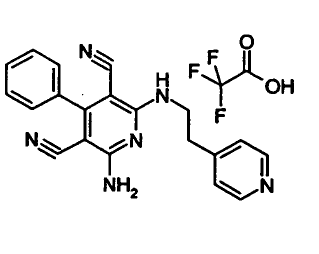 | 340 | 341 | 68 |
| 29 | 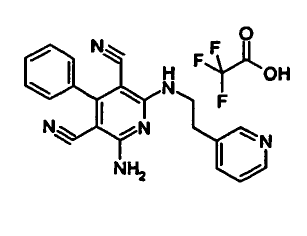 | 340 | 341 | 82 |
| 30 | 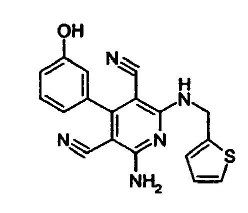 | 347 | 348 | 60 |
| 31 | 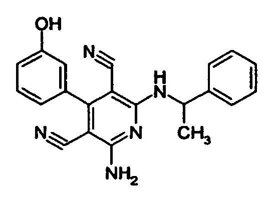 | 355 | 356 | 67 |
| 32 | 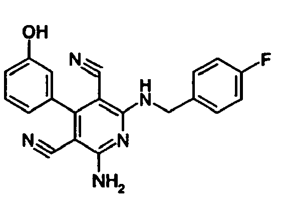 | 359 | 360 | 73 |
| 33 | 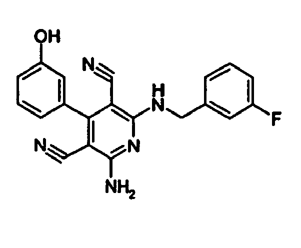 | 359 | 360 | 85 |
| 34 | 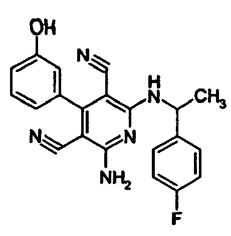 | 373 | 374 | 72 |
| 35 | 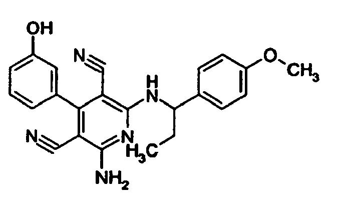 | 399 | 400 | 59 |
| 36 | 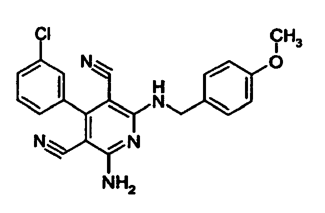 | 390 | 391 | 22 |
| 37 | 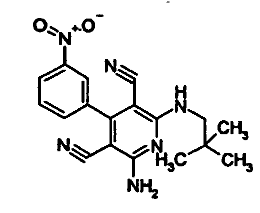 | 350 | 351 | 28 |
| 38 | 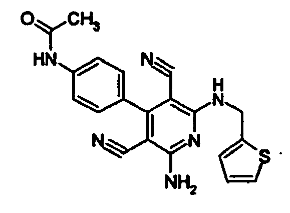 | 388 | 389 | 87 |
| 39 | 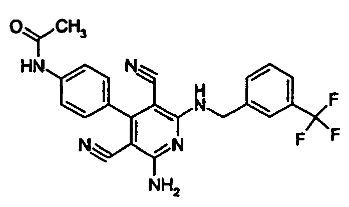 | 450 | 451 | quantitativ |
| 40 | 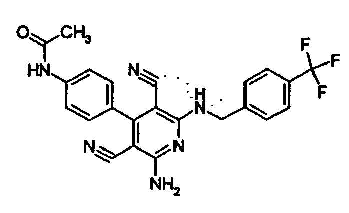 | 450 | 451 | 77 |

### Beispiel 3 zeigt folgende ¹H-NMR-spektroskopische Daten:

¹H-NMR-Spektrum [DMSO-d₆]: δ = 4,55 [2H] d; 6,8 [1H] s; 6,85 [1H] d; 6,9 [1H] d; 7,3 [1H] tr; 7,3-7,5 [2H] m breit; 7,95 [1H] s; 8,1 [1H] tr; 8,45 [1H] s; 8,8 [1H] s; 9,8 [1H] s breit.

### Beispiel 4 zeigt folgende ¹H-NMR-spektroskopische Daten:

¹H-NMR-Spektrum [DMSO-d₆]: δ = 4,55 [2H] d; 6,8 [1H] s; 6,85 [1H] dd; 6,9 [1H] d; 7,2-7,45 [8H] m; 8,0 [1H] tr; 9,8 [1H] s.

### Beispiel 41

### 1. Stufe

### 2-Chlor-4-phenyl-6-(phenylsulfanyl)-3,5-pyridindicarbonitril

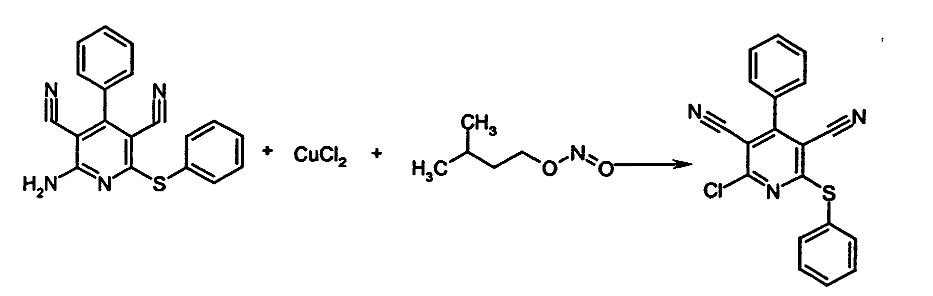

24,5 g (182 mmol) Kupfer(II)chlorid wasserfrei werden in 180 ml Acetonitril unter Argon suspendiert, dann werden 21,4 g (182 mmol) Isopentylnitrit zugetropft. Nach 20 min Rühren bei Raumtemperatur werden 10 g (30,5 mmol) 2-Amino-4-phenyl-6-(phenyl-sulfanyl)-3,5-pyridindicarbonitril [Kambe et al., Synthesis, 531-533 (1981)] zugegeben. Es wird über das Wochenende bei Raumtemperatur gerührt. Zur Aufarbeitung wird der Ansatz mit 150 ml 1 N Salzsäure versetzt, 4 x mit Dichlormethan extrahiert, die organische Phase wird einmal mit gesättigter Kochsalz-Lösung gewaschen, über Magnesiumsulfat getrocknet und im Vakuum eingedampft. Der Rückstand wird mit wenig Ethylacetat verrührt und 1 Stunde im Kühlschrank stehen gelassen. Die Kristalle werden abgesaugt und mit wenig kaltem Ethylacetat und Diethylether gewaschen.
Ausbeute: 7,26 g (68,5 % d.Th.) Produkt
Massenspektrum: gesuchte Molmasse: 347, gefunden [M+H]⁺=348

### 2. Stufe

### 2-[(2-Hydroxyethyl)amino]-4-phenyl-6-(phenylsulfanyl)-3,5-pyridindicarbonitril

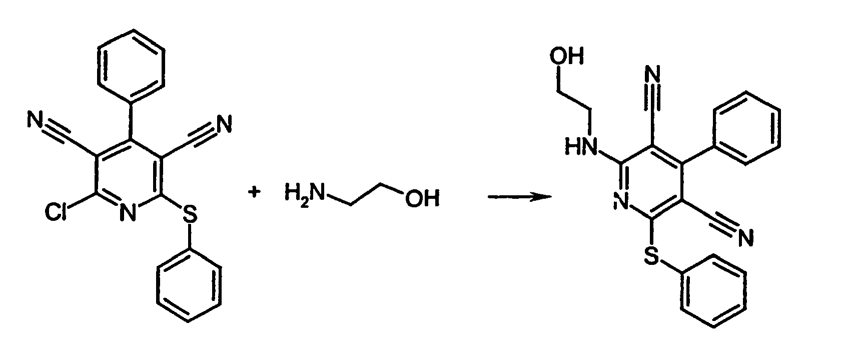

0,7 g (2 mmol) 2-Chlor-4-phenyl-6-(phenylsulfanyl)-3,5-pyridindicarbonitril (1. Stufe) werden in 2 ml Dimethylformamid (DMF) unter Argon gelöst, mit 0,27 g (4,5 mmol) 2-Hydroxyethylamin versetzt und 15 Minuten bei Raumtemperatur gerührt. Nach Zugabe von 2 ml Methanol wird ca. 1 ml Wasser zugetropft und der Niederschlag abgesaugt. Die Kristalle werden in 4 ml Methanol ausgekocht.
Ausbeute: 410 mg (= 55 % d.Th.) Produkt
Massenspektrum: gesuchte Molmasse: 372, gefunden [M+H]⁺=373

### 3. Stufe

### 2-(Benzylamino)-6-[(2-hydroxyethyl)amino]-4-phenyl-3,5-pyridindicarbonitril

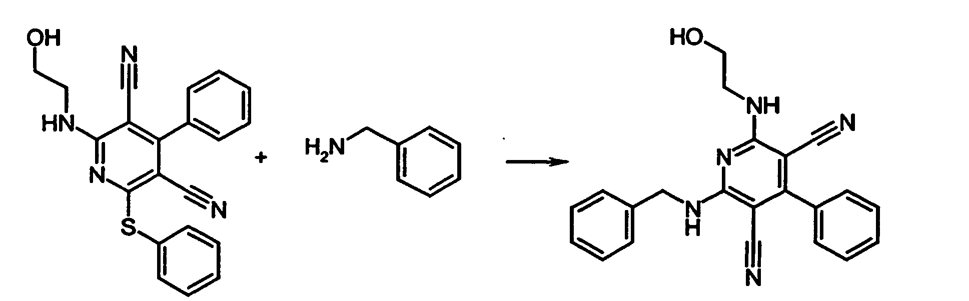

32,4 mg (0,1 mmol) 2-[(2-Hydroxyethyl)amino]-4-phenyl-6-(phenylsulfanyl)-3,5-pyridindicarbonitril (2. Stufe) werden zusammen mit 43 mg (0,4 mmol) Benzylamin in 0,5 ml Dimethylsulfoxid (DMSO) 2 Stunden bei 120°C geschüttelt. Die Reaktionslösung wird durch präparative HPLC in zwei Injektionen gereinigt.
Säule: Kromasil 100 C18 5µm 20x50 mm,
Vorsäule: Gromsil ODS 4 HE 15µm 10x20 mm.
Flußrate: 25 ml/min.
Gradient (A = Acetonitril, B=Wasser + 0.3% Trifluoressigsäure):

| | |
|---|---|
| 0 min | 10% A ; |
| 2.00 min | 10% A ; |
| 6.00 min | 90% A ; |
| 7.00 min | 90% A ; |
| 7.10 min | 10% A; |
| 8 min | 10% A. |

Detektion: 220 nm. Injektionsvolumen: 300 µl DMSO-Lsg.
Die Produktfraktion wird im Vakuum eingedampft.
Ausbeute: 35,3 mg (= 47,8 % d.Th.) Produkt
Massenspektrum: gesuchte Molmasse: 369, gefunden [M+H]⁺=370

Die in der folgenden Tabelle aufgeführten Verbindungen (Beispiel 42 bis 59) werden analog zu den zuvor aufgeführten Vorschriften des Beispiels 41 hergestellt. Die Identität und Reinheit der Verbindungen wird durch LC-MS nachgewiesen.

| **Bsp.-Nr.** | **Zielstruktur** | **gesuchte Molmasse** | **gefunden [M+H]⁺** | **Ausbeute (% d.Th.)** |
|---|---|---|---|---|
| 42 | 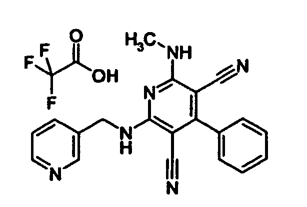 | 454 | 455 | 67 |
| 43 | 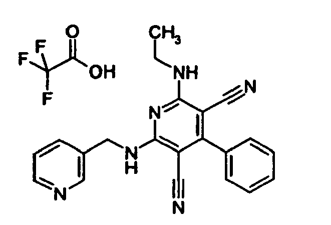 | 468 | 469 | 54 |
| 44 | 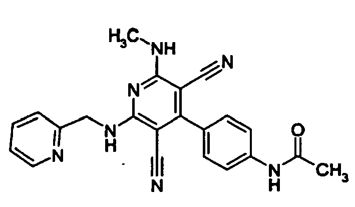 | 397 | 398 | 77 |
| 45 | 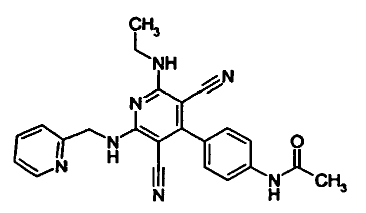 | 411 | 412 | 70 |
| 46 | 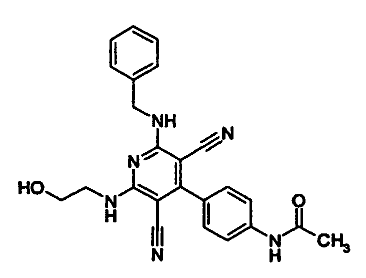 | 426 | 427 | 68 |
| 47 | 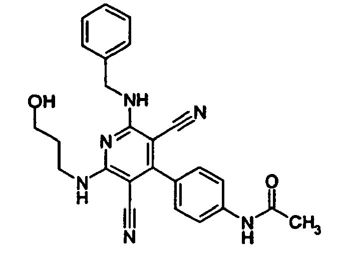 | 441 | 442 | 33 |
| 48 | 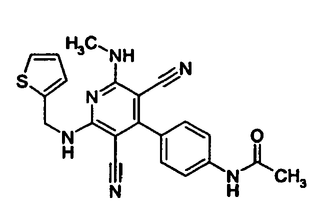 | 402 | 403 | 48 |
| 49 | 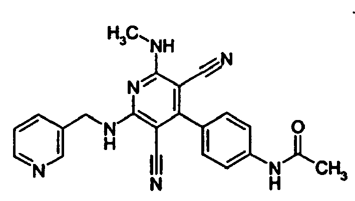 | 397 | 398 | 40 |
| 50 | 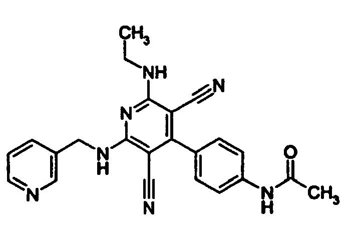 | 411 | 412 | 55 |
| 51 | 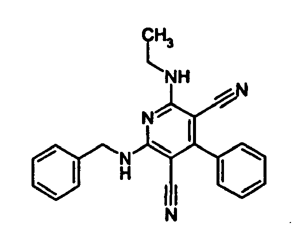 | 353 | 354 | 40 |
| 52 | 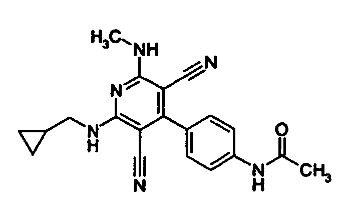 | 360 | 361 | 56 |
| 53 | 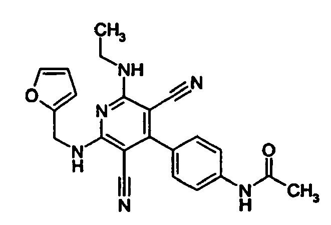 | 400 | 401 | 51 |
| 54 | 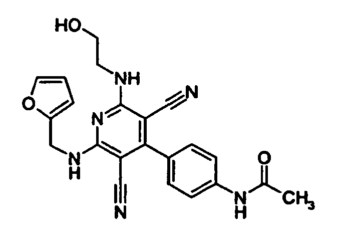 | 416 | 417 | 33 |
| 55 | 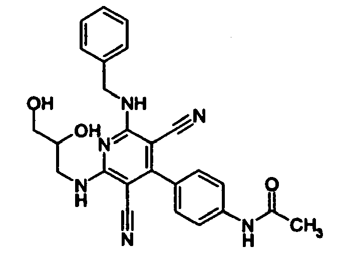 | 457 | 458 | 54 |
| 56 | 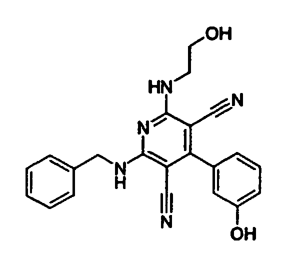 | 385 | 386 | 68 |
| 57 | 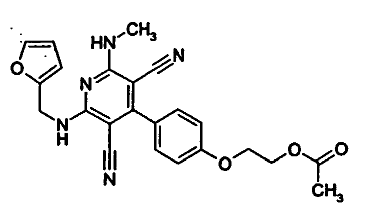 | 431 | 432 | 99 |
| 58 | 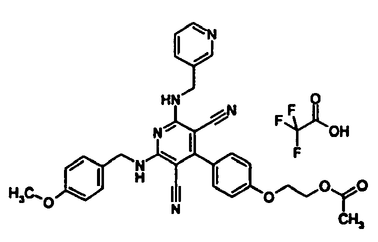 | 548 | 549 | 42 |
| 59 | 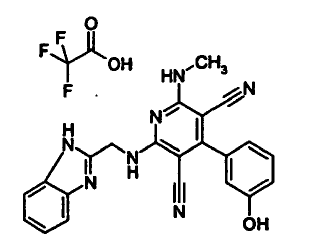 | 395 | 396 | 46 |

### Beispiel 60

### 2-Amino-4-(2,3-dihydro-1,4-benzodioxin-6-yl)-6-[(3-pyridinylmethyl)amino]-3,5-pyridindicarbonitril

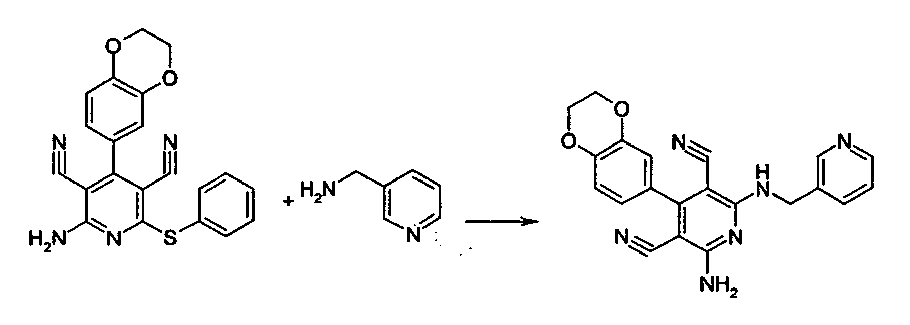

100 mg (0,26 mmol) 2-Amino-4-(2,3-dihydro-1,4-benzodioxin-6-yl)-6-(phenylsulfanyl)-3,5-pyridindicarbonitril werden zusammen mit 56 mg (0,52 mmol) 3-Picolylamin in 8 ml DMF auf 100°C erhitzt. Nach 3 Stunden werden noch einmal 224 mg (2,08 mmol) 3-Picolylamin zugegeben und es wird weitere 4 Stunden auf 100 °C erhitzt. Nach Verdünnen mit Wasser wird dreimal mit Ethylacetat extrahiert. Die vereinigten organischen Phasen werden mit gesättigter Kochsalzlösung gewaschen, über Magnesiumsulfat getrocknet und eingedampft. Der Eindampfrückstand wird durch präparative HPLC an Reversed Phase Kieselgel gereinigt.
Die Produktfraktion wird eingedampft.
Ausbeute: 31,3 mg (= 31 % d.Th.) Produkt
Massenspektrum: gesuchte Molmasse: 384, gefunden [M+H]⁺=385
¹H-NMR-Spektrum [DMSO-d₆]: δ = 4,3 [4H] s; 4,55 [2H] d; 6,85 - 7,0 (3H] m; 7,3 - 7,55 [3H] m; 7,85 [1H] d; 8,1 [1H] tr; 8,45 [1H] d; 8,65 [1H] d.

### Beispiel 61

### 2-Amino-4-(2,3-dihydro-1,4-benzodioxin-6-yl)-6-[(2-pyridinylmethyl)amino]-3,5-pyridindicarbonitril

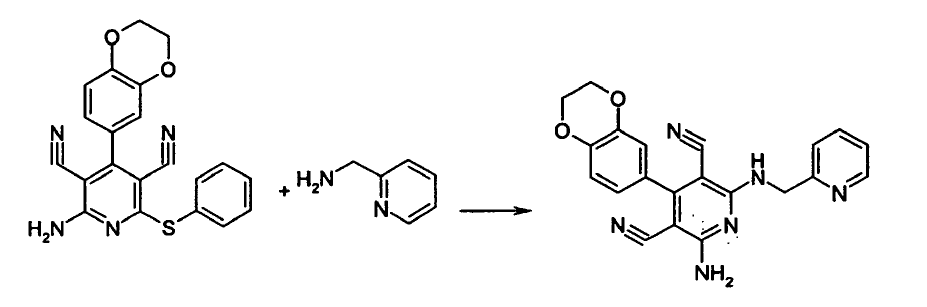

100 mg (0,26 mmol) 2-Amino-4-(2,3-dihydro-1,4-benzodioxin-6-yl)-6-(phenylsulfanyl)-3,5-pyridindicarbonitril werden zusammen mit 56 mg (0,52 mmol) 2-Picolylamin in 8 ml DMF auf 100°C erhitzt. Nach 3 Stunden werden noch einmal 224 mg (2,08 mmol) 2-Picolylamin zugegeben und es wird weitere 4 Stunden auf 100 °C erhitzt. Nach Verdünnen mit Wasser wird dreimal mit Ethylacetat extrahiert. Die vereinigten organischen Phasen werden mit gesättigter Kochsalzlösung gewaschen, über Magnesiumsulfat getrocknet und eingedampft. Der Eindampfrückstand wird durch präparative HPLC an Reversed Phase Kieselgel gereinigt.
Die Produktfraktion wird eingedampft und noch einmal durch präparative Dünnschichtchromatographie (Laufmittel Toluol/Ethylacetat 1:1) gereinigt.
Ausbeute: 2 mg (= 2 % d.Th.) Produkt
Massenspektrum: gesuchte Molmasse: 384, gefunden [M+H]⁺=385
¹H-NMR-Spektrum [DMSO-d₆]: δ = 4,3 [4H] s; 4,7 [2H] d; 6,9 - 7,05 (3H] m; 7,2 - 7,4 [4H] m; 7,75 [1H] tr; 7,9 [1H] tr; 8,5 [1H] d.

### Beispiel 62

### 2-Amino-4-(2,3-dihydro-1,4-benzodioxin-6-yl)-6-{[(2-methyl-1,3-thiazol-4-yl)-methyl]amino}-3,5- pyridindicarbonitril

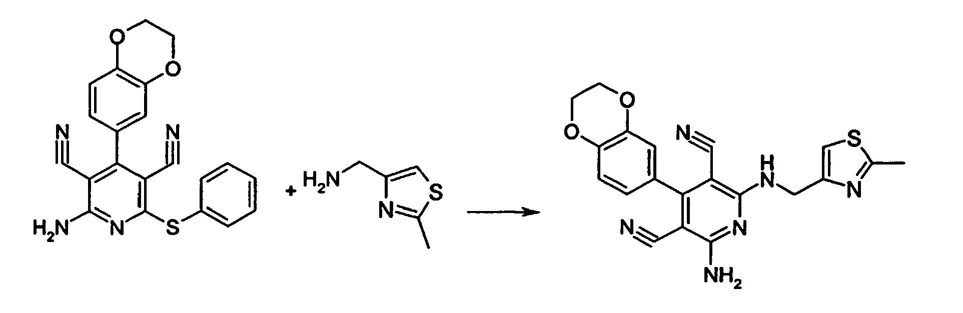

100 mg (0,26 mmol) 2-Amino-4-(2,3-dihydro-1,4-benzodioxin-6-yl)-6-(phenylsulfanyl)-3,5-pyridindicarbonitril werden zusammen mit 66 mg (0,52 mmol) (2-Methyl-1,3-thiazol-4-yl)methylamin in 8 ml 1,2-Dimethoxyethan auf 90°C erhitzt. Nach 2,5 Stunden werden noch einmal ca 500 mg (ca 3,9 mmol) ) (2-Methyl-1,3-thiazol-4-yl)methylamin zugegeben und es wird weitere 24 Stunden auf 90 °C erhitzt. Das Reaktionsgemisch wird eingedampft und der Eindampfrückstand durch präparative HPLC an Reversed Phase Kieselgel gereinigt.
Die Produktfraktion wurde eingedampft.
Ausbeute: 60 mg (= 52 % d.Th.) Produkt
Massenspektrum: gesuchte Molmasse: 404, gefunden [M+H]⁺=405
¹H-NMR-Spektrum [DMSO-d₆]: δ = 2,65 [3H] s; 4,3 [4H] s; 4,65 [2H] s; 7,0 (3H] m; 7,3 [1H] s; 7,4 [2H] s breit; 7,9 [1H] s breit.

## Patentansprüche

1. Verbindungen der Formel (I) 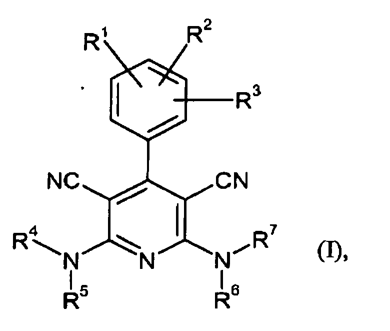 worin
R¹ Wasserstoff, Hydroxy, Chlor, Nitro, Methyl, Methoxy, Ethoxy, n-Propoxy, Isopropoxy, n-Butoxy oder -NH-C(O)-CH₃ bedeuten, wobei die Alkoxyreste ihrerseits durch Hydroxy, Methoxy, Ethoxy, n-Propoxy, Isopropoxy, n-Butoxy, -O-C(O)-CH₃ oder Cyclopropyl substituiert sein können,
R² und R³ Wasserstoff bedeuten,
R⁴ Wasserstoff, Methyl, Ethyl, n-Propyl oder Isopropyl bedeutet, wobei die Alkylreste ihrerseits durch Pyridyl, Methoxy, Ethoxy, n-Propoxy, Isopropoxy, n-Butoxy oder ein- oder zweifach durch Hydroxy substituiert sein können,
R⁵ Wasserstoff bedeutet,
R⁶ Methyl, Ethyl n-Propyl, Isopropyl, wobei die Alkylreste ihrerseits ein- oder zweifach, unabhängig voneinander, durch Cyclopropyl, Phenyl, das seinerseits wiederum durch Fluor, Trifluormethyl oder Methoxy substituiert sein kann, Pyridyl, Furyl, Thienyl, Benzimidazolyl, Pyrrolidinonyl, N-Methyl-Pyrrolidinonyl, N-Methyl-Pyrrolidinyl, N-Ethyl-Pyrrolidinyl, N-Methyl-Imidazolidinonyl substituiert sein können, oder Cyclopropyl bedeutet
und
R⁷ Wasserstoff bedeutet
und ihre Salze, Hydrate, Hydrate der Salze und Solvate
ausgenommen die Verbindung 2-Amino-4-phenyl-6-[(phenylmethyl)amino]-3,5-pyridindicarbonitril.

2. Verfahren zur Herstellung von Verbindungen der Formel (I), wie in Anspruch 1 definiert, **dadurch gekennzeichnet, dass** man
Verbindungen der Formel (II) 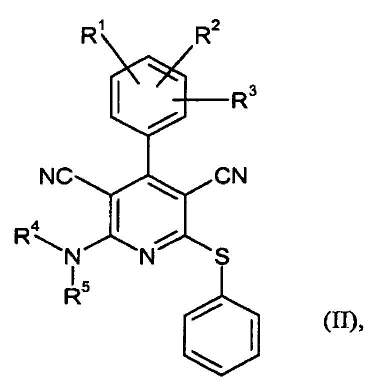 in welcher
R¹, R², R³, R⁴ und R⁵ die in Anspruch 1 angegebene Bedeutung haben,
in einem Lösemittel mit Verbindungen der Formel (III)
R⁶-NH-R⁷ (III),
in welcher
R⁶ und R⁷ die in Anspruch 2 angegebene Bedeutung haben,
umsetzt.

3. Verbindungen der Formel (I), wie in Anspruch 1 definiert, zur Prophylaxe und/oder Behandlung von Erkrankungen.

4. Zusammensetzung, enthaltend mindestens eine Verbindungen der Formel (I), wie in Anspruch 1 definiert, und mindestens einen weiteren Hilfsstoff.

5. Verwendung von Verbindungen der Formel (I), wie in Anspruch 1 definiert, zur Herstellung von Arzneimitteln zur Prophylaxe und/oder Behandlung von Erkrankungen des Herzkreislaufsystems (kardiovaskulären Erkrankungen).

6. Verwendung von Verbindungen der Formel (I), wie in Anspruch 1 definiert, zur Herstellung von Arzneimitteln zur Prophylaxe und/oder Behandlung von Diabetes.

## Claims

1. Compounds of the formula (I) 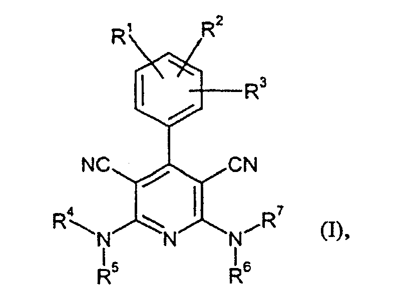 in which
R¹ represents hydrogen, hydroxyl, chlorine, nitro, methyl, methoxy, ethoxy, n-propoxy, isopropoxy, n-butoxy or -NH-C(O)-CH₃, where the alkoxy radicals for their part may be substituted by hydroxyl, methoxy, ethoxy, n-propoxy, isopropoxy, n-butoxy, -O-C(O)-CH₃ or cyclopropyl,
R² and R³ represent hydrogen,
R⁴ represents hydrogen, methyl, ethyl, n-propyl or isopropyl, where the alkyl radicals for their part may be substituted by pyridyl, methoxy, ethoxy, n-propoxy, isopropoxy, n-butoxy or mono- or disubstituted by hydroxyl,
R⁵ represents hydrogen,
R⁶ represents methyl, ethyl, n-propyl, isopropyl, where the alkyl radicals for their part may be mono- or disubstituted, independently of one another, by cyclopropyl, phenyl which for its part may be substituted by fluorine, trifluoromethyl or methoxy, pyridyl, furyl, thienyl, benzimidazolyl, pyrrolidinonyl, N-methylpyrrolidinonyl, N-methylpyrrolidinyl, N-ethylpyrrolidinyl, N-methylimidazolidinonyl, or cyclopropyl
and
R⁷ represents hydrogen
and the salts, hydrates, hydrates of the salts and solvates thereof
with the exception of the compound 2-amino-4-phenyl-6-[(phenylmethyl)amino]-3,5-pyridinedicarbonitrile.

2. Process for preparing compounds of the formula (I) as defined in claim 1, **characterized in that**
compounds of the formula (II) 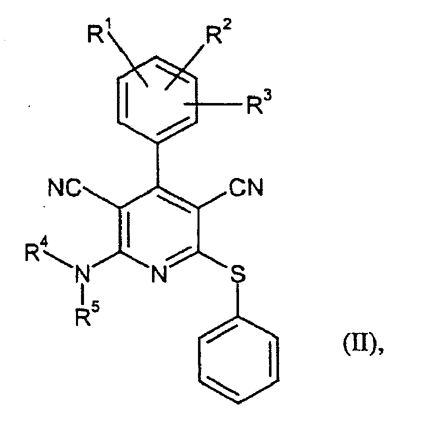 in which
R¹, R², R³, R⁴ and R⁵ are as defined in claim 1,
are reacted in a solvent with compounds of the formula (III)
R⁶-NH-R⁷ (III)
in which
R⁶ and R⁷ are as defined in claim 1.

3. Compounds of the formula (I) as defined in claim 1 for the prophylaxis and/or treatment of disorders.

4. Composition, comprising at least one compound of the formula (I) as defined in claim 1 and at least one auxiliary.

5. Use of compounds of the formula (I) as defined in claim 1 for preparing medicaments for the prophylaxis and/or treatment of disorders of the cardiovascular system (cardiovascular disorders).

6. Use of compounds of the formula (I) as defined in claim 1 for preparing medicaments for the prophylaxis and/or treatment of diabetes.

## Revendications

1. Composés de formule (I) 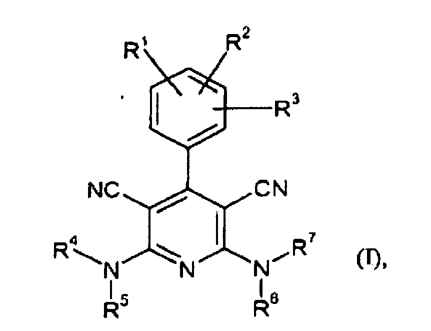 dans laquelle
R¹ représente l'hydrogène, un groupe hydroxy, le chlore, un groupe nitro, un reste méthyle, méthoxy, éthoxy, n-propoxy, isopropoxy, n-butoxy ou -NH-C(O)-CH₃, les restes alkoxy pouvant eux-mêmes être substitués par un radical hydroxy, méthoxy, éthoxy, n-propoxy, isopropoxy, n-butoxy, -O-C(O)-CH₃ ou cyclopropyle,
R² et R³ représentent l'hydrogène,
R⁴ représente l'hydrogène, un reste méthyle, éthyle, n-propyle ou isopropyle, les restes alkyle pouvant eux-mêmes être substitués par un radical pyridyle, méthoxy, éthoxy, n-propoxy, isopropoxy, n-butoxy, ou substitués une ou deux fois par un radical hydroxy,
R⁵ représente l'hydrogène,
R⁶ représente un reste méthyle, éthyle, n-propyle, isopropyle, les restes alkyle pouvant eux-mêmes être substitués une ou deux fois indépendantes l'une de l'autre par un radical cyclopropyle, phényle qui peut lui-même être substitué par un radical fluoro, trifluorométhyle ou méthoxy, pyridyle, furyle, thiényle, benzimidazolyle, pyrrolidinonyle, N-méthylpyrrolidinonyle, N-méthyl-pyrrolidinyle, N-éthyl-pyrrolidinyle, N-méthyl-imidazolidinonyle, ou un reste cyclopropyle,
et
R⁷ représente l'hydrogène et leurs sels, leurs hydrates, les hydrates de leurs sels et leurs produits de solvatation, excepté le composé 2-amino-4-phényl-6-[(phénylméthyl)amino]-3,5-pyridinedicarbonitrile.

2. Procédé de production de composés de formule (I) telle que définie dans la revendication 1, **caractérisé en ce qu'**on fait réagir dans un solvant des composés de formule (II) 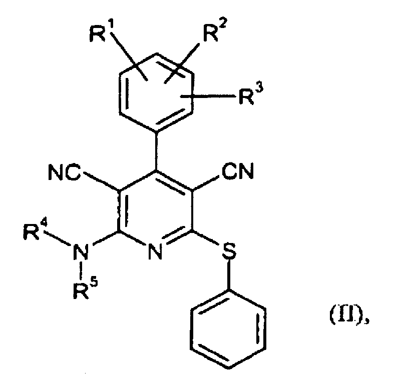 dans laquelle
R¹, R², R³, R⁴ et R⁵ ont la définition indiquée dans la revendication 1, avec des composés de formule (III)
R⁶-NH-R⁷ (III)
dans laquelle
R⁶ et R⁷ ont la définition indiquée dans la revendication 2.

3. Composés de formule (I) telle que définie dans la revendication 1, destinés à la prophylaxie et/ou au traitement de maladies.

4. Composition contenant au moins un composé de formule (I) telle que définie dans la revendication 1 et au moins une autre substance auxiliaire.

5. Utilisation de composés de formule (I) telle que définie dans la revendication 1 pour la préparation de médicaments destinés à la prophylaxie et/ou au traitement de maladies du système cardio-vasculaire (maladies cardio-vasculaires).

6. Utilisation de composés de formule (I) telle que définie dans la revendication 1 pour la préparation de médicaments destinés à la prophylaxie et/ou au traitement du diabète.
